# EUROPÄISCHE PATENTANMELDUNG

(11) **EP 4 810 471 A1**
(43) Veröffentlichungstag der Anmeldung: **23.09.2026**
(21) Anmeldenummer: 25164644.4
(22) Anmeldetag: 19.03.2025
(51) Int. Cl.: C07C 45/50, C07C 47/02, C07F 9/6574, C07F 15/00

(54) **PHOSPHITE MIT DI-BROMO-BISNAPHTHALIN-RÜCKGRAT UND FLÜGEL**

(71) Anmelder: Evonik Oxeno GmbH & Co. KG, 45772 Marl (DE)
(72) Erfinder: FRANKE, Robert, 45772 Marl (DE); BÜKER, Julia, 44803 Bochum (DE); SALE, Anna Chiara, 40474 Düsseldorf (DE); BÖRNER, Armin, 18059 Rostock (DE); HOLZ, Jens, 18196 Kessin (DE); ROMEIKE, Kerstin, 18109 Rostock (DE); WENZEL, Gudrun, 18196 Kessin (DE)
(74) Vertreter: Evonik Patent Association

(57) **Zusammenfassung**

Die Erfindung betrifft Verbindungen in Form von Organobisphosphiten der Formeln (I-i) bis (I-vii). Die Erfindung betrifft zudem die Verwendung der Verbindungen als Ligand eines Katalysators in einer Hydroformylierungsreaktion. Die vorliegende Erfindung betrifft zudem eine Substanz umfassend eine Verbindung der Formeln (I-i) bis (I-vii) und die Verwendung dieser Substanz als Katalysator in einer Hydroformylierungsreaktion. Die vorliegende Erfindung betrifft darüber hinaus ein katalytisches Verfahren zur Herstellung von Aldehyden.

## Beschreibung

Die vorliegende Erfindung betrifft eine Verbindung gemäß den Formeln (I-i) bis (I-vii) und die Verwendung der Verbindung als Ligand eines Katalysators in einer Hydroformylierungsreaktion. Die vorliegende Erfindung betrifft zudem eine Substanz und die Verwendung dieser Substanz als Katalysator in einer Hydroformylierungsreaktion. Die vorliegende Erfindung betrifft darüber hinaus ein Verfahren zur Herstellung von Aldehyden.

Die Hydroformylierung (auch Oxosynthese oder Roelen-Reaktion genannt) war die erste großtechnisch genutzte und heutzutage mit über 10 Mio. t/a mengenmäßig wichtigste Synthese, welche mit metallorganischen Katalysatoren durchgeführt wurde/wird. Bei der Hydroformylierung reagiert ein Synthesegas aus Wasserstoff und Kohlenstoffmonoxid mit Kohlenstoff-Doppelbindungen von Olefinen unter Bildung von Aldehyden; die Reaktion wird vorzugsweise durch Cobalt- oder Rhodiumkatalysatoren katalysiert.

Durch die Auswahl der Komponenten, insbesondere durch elektronische und sterische Eigenschaften von Metallatom und Liganden des metallorganischen Katalysators, kann die Hydroformylierung von Olefinen gesteuert und optimiert werden. Insbesondere kann durch die gezielte Auswahl der Liganden in metallorganischen Katalysatoren der Umsatz und auch die Selektivität gesteigert werden.

Als Liganden des Katalysators spielen insbesondere die Organobisphosphite eine herausragende Rolle (siehe R. Franke, D. Selent, A. Börner Chem. Rev. 2012, 112, 11, 5675-5732, https://doi.org/10.1021/cr3001803).

Die als Ligand eingesetzten Verbindungen sollen hierbei insbesondere einen hohen Umsatz der Olefine in Aldehyde gewährleisten.

Demnach liegt der vorliegenden Erfindung die primäre Aufgabe zu Grunde, neue Verbindungen herzustellen, die als Liganden in Katalysatorsystemen für die Hydroformylierung geeignet sind.

Entsprechend basiert die vorliegende Erfindung auch auf der Aufgabe, eine Substanz enthaltend die zuvor genannten Verbindungen anzugeben, welche katalytisch aktiv ist.

Die vorliegende Erfindung basiert zudem auf der Aufgabe, ein (katalytisches) Verfahren zur Herstellung von Aldehyden aus Olefinen anzugeben, wobei insbesondere der Umsatz optimiert werden soll.

Diese Aufgaben sind eng verknüpft mit der ergänzenden Aufgabenstellung, eine Verwendung für die zuvor genannten Verbindungen und/oder die zuvor genannten Substanzen anzugeben.

Weitere Aufgaben ergeben sich aus der nachfolgenden Beschreibung und den Patentansprüchen.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele. Soweit für einen erfindungsgemäßen Aspekt (Verbindung; Substanz; Verfahren zur Herstellung von Aldehyden; Verwendung einer erfindungsgemäßen Verbindung als Ligand eines Katalysators, Verwendung einer erfindungsgemäßen Substanz als Katalysator) bestimmte Ausgestaltungen als bevorzugt bezeichnet werden, gelten die entsprechenden Ausführungen jeweils auch für die anderen Aspekte der vorliegenden Erfindung, *mutatis mutandis.* Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Der Gegenstand der vorliegenden Erfindung wird in den beigefügten Patentansprüchen und der vorliegenden Beschreibung definiert.

Der hierin verwendete Begriff "Ligand" hat die in der Komplexchemie übliche Bedeutung und bezeichnet ein Atom oder Molekül, welches sich über eine koordinative Bindung an ein zentrales Metallatom bzw. im Falle von verbrückenden und chelatisierenden Liganden an mehrere Metallatome binden kann.

Der hierin verwendete Begriff "Katalysator" hat die übliche Bedeutung und wird für Substanzen verwendet, die die Aktivierungsenergie einer bestimmten Reaktion herabsetzen und dadurch die Reaktionsgeschwindigkeit erhöhen, ohne bei der Reaktion verbraucht zu werden und ohne im Endprodukt zu erscheinen. Katalysatoren gehen definitionsgemäß unverändert aus der Reaktion hervor.

Die hierin beschriebenen Katalysatoren sind bevorzugt Metallkomplexe, die eines oder mehrere Metallatome (in Komplexen auch oft als "Zentralatom" bezeichnet) und einen oder mehrere Liganden enthalten.

Der hierin verwendete Begriff "Selektivität" bezeichnet den prozentualen Anteil eines spezifischen Produkts in einem Gemisch aus mehreren möglichen Produkten. Werden beispielsweise 100 Moleküle A als eingesetzter Ausgangsstoff sowohl in 25 Moleküle B als auch in 25 Moleküle C und 50 Moleküle D umgesetzt, so beträgt die Selektivität für die Moleküle B und C jeweils 25%, während die Selektivität für das Molekül D 50% beträgt.

Die vorliegende Erfindung betrifft insbesondere eine Verbindung, wobei die Verbindung ein Organobisphosphit ist, ausgewählt aus den folgenden Formeln (I-i) bis (I-vii) 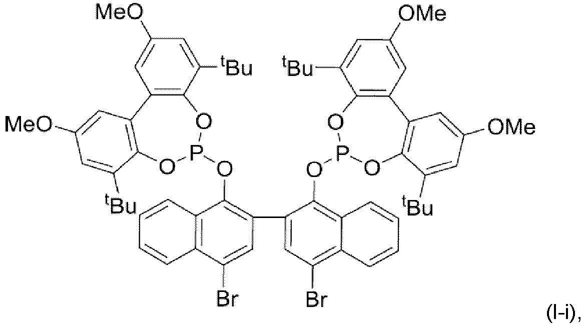 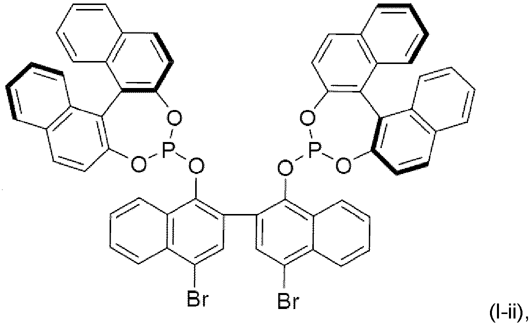 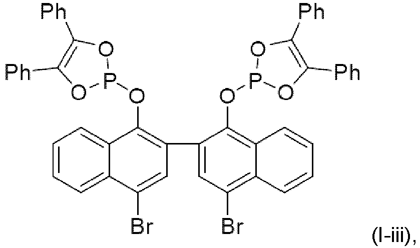 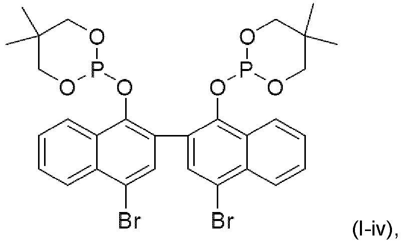 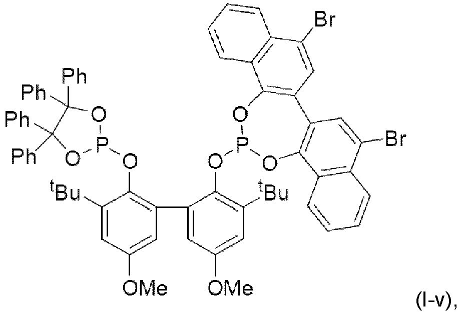 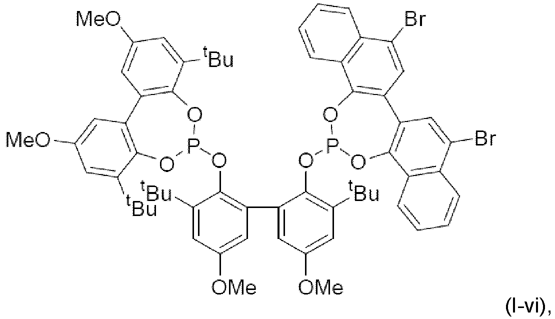 oder

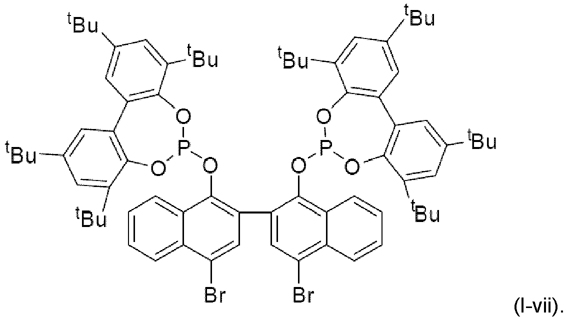

Die erfindungsgemäßen Verbindungen konnten erstmalig synthetisiert werden und haben sich in eigenen Untersuchungen als besonders vorteilhafte Liganden zur katalytischen Herstellung von Aldehyden aus Olefinen in einer Hydroformylierungsreaktion herausgestellt.

Die erfindungsgemäßen Verbindungen gemäß Formeln (I-i) bis (I-vii) sind Organobisphosphite (oft auch als "Bisphosphite" bezeichnet). Ein Phosphit hat die allgemeine Form P(OR)₃ mit (R = Alkyl-Rest oder Aryl-Rest). Durch geschickte Wahl des Bindeglieds der beiden Phosphitgruppen und durch weiterhin geschickte Wahl von verbrückten Substituenten der Phosphitgruppen konnten im Rahmen der vorliegenden Erfindung für die Hydroformylierung besonders gut geeignete Liganden bereitgestellt werden. Die erfindungsgemäßen Verbindungen gemäß Formeln (I-i) bis (I-vii) weisen zwei potenzielle Bindungsstellen zu einem Metallzentralatom auf, liegen also in Form zweizähniger Liganden vor, was die daraus resultierende Metallkomplexe durch den sogenannten Chelateffekt besonders stabil und robust macht.

Die im Rahmen der vorliegenden Erfindung durchgeführten Untersuchungen, vgl. auch die unten gezeigten Beispiele, zeigen, dass mit den erfindungsgemäßen Organobisphosphit-Liganden deutlich bessere Umsätze bei der Synthese von Aldehyden aus Olefinen in einer Hydroformylierungsreaktion erzielt werden können, als mit bekannten Systemen, insbesondere bessere Umsätze als mit Organobisphosphinit-Liganden (oft auch als "Bisphosphinit" bezeichnet).

Des Weiteren zeigen die mit diesen Liganden hergestellten Katalysatoren die vorteilhafte Eigenschaft interne Olefine (zum Beispiel 2-Penten) in einem nicht unerheblichen Anteil zu terminalen Aldehyden zu hydroformylieren, sogenannte n-Selektivität.

Die vorliegende Erfindung betrifft zudem eine Substanz, wobei die Substanz
i) eine Mischung ist, die Mischung umfassend
   - eine oder mehrere erfindungsgemäße Verbindungen,
      und
   - eines oder mehrere Metallatome,
   und/oder
ii) ein Metallkomplex ist oder einen Metallkomplex enthält, der Metallkomplex umfassend
   - eine oder mehrere erfindungsgemäße Verbindungen,
      und
   - eines oder mehrere Metallatome.

Erfindungsgemäße Substanzen in Form von Mischungen oder Metallkomplexen wie vorstehend und nachstehend definiert eignen sich insbesondere als Katalysatorsysteme in Hydroformylierungsreaktionen.

In erfindungsgemäßen Substanzen sind neben den erfindungsgemäßen Verbindungen zusätzlich eines oder mehrere Metallatome umfasst. Die erfindungsgemäßen Verbindungen, d.h. die Liganden und die Metallatome (vor der Komplexbildung oft auch als "Metallprecursor" bezeichnet) bilden bevorzugt einen Metallkomplex, der katalytisch aktiv ist, insbesondere in einer Hydroformylierungsreaktion.

Der Metallkomplex kann *in situ* durch Zusammengeben des erfindungsgemäßen Liganden und eines Metallprecursors erzeugt werden (Variante i).

Der Metallkomplex mit dem entsprechenden Liganden kann jedoch auch gesondert hergestellt, isoliert und weiterverwendet, insbesondere als Katalysator eingesetzt werden, oder an Dritte verkauft werden (Variante ii).

Bevorzugt ist eine erfindungsgemäße Substanz, wobei das molare Verhältnis von Metallatomen zu Verbindungen gemäß Formeln (I-i) bis (I-vii), d.h. den Liganden, in der Mischung und/oder im Metallkomplex im Bereich von 0,5:1 bis 1:4 liegt, bevorzugt im Bereich von 0,9:1 bis 1:2,1 liegt, besonders bevorzugt 1:1 oder 1:2 ist.

Eine solche erfindungsgemäße Substanz in Form einer Mischung oder eines Metallkomplexes eignet sich insbesondere als Katalysator zur Hydroformylierung. Die zuvor angegebenen bevorzugten molaren Verhältnisse von Metallatomen zu Verbindungen gemäß Formeln (I-i) bis (I-vii) erlauben hierbei eine besonders effiziente

Ebenfalls bevorzugt ist eine erfindungsgemäße Substanz, wobei das eine oder eines der mehreren Metallatome in der Mischung und/oder im Metallkomplex ausgewählt ist aus der Gruppe bestehend aus Rhodium, Cobalt, Eisen, Ruthenium, Osmium, Iridium, Platin und Zinn, bevorzugt ist das eine oder eines der mehreren Metallatome Rhodium.

Bevorzugt ist das eine oder eines der mehreren Metallatome im Rahmen der vorliegenden Erfindung Cobalt oder Rhodium. Cobalt und Rhodium haben sich als Metallatom in Katalysatoren für die Hydroformylierung als besonders geeignet erwiesen.

Ebenfalls bevorzugt ist eine erfindungsgemäße Substanz in Form einer Mischung, wobei die Mischung als weiteren Bestandteil umfasst
- ein oder mehrere Olefine mit jeweils einer oder mehreren Kohlenstoff-Doppelbindungen, bevorzugt ausgewählt aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
   und/oder (bevorzugt "und)
- Lösungsmittel, wobei das Lösungsmittel vorzugsweise Toluol umfasst oder Toluol ist.

Vorgenannte bevorzugte Ausgestaltungen der erfindungsgemäßen Substanz in Form einer Mischung, zusätzlich umfassend eines oder mehrere Olefine mit jeweils einer oder mehreren Kohlenstoff-Doppelbindungen können direkt in einer Hydroformylierungsreaktion eingesetzt werden. Sie umfassen sowohl einen wie zuvor beschriebenen Katalysator als auch eine oder mehrere olefinische Ausgangsverbindungen. Bevorzugt umfasst die Mischung ebenfalls ein geeignetes Lösungsmittel, insbesondere Toluol, um die Reaktionsführung zu erleichtern.

In einer Hydroformylierungsreaktion findet formal die Addition eines Wasserstoffs (-H) und einer Formylgruppe (-CHO) an eine Kohlenstoff-Doppelbindung eines Moleküls statt; somit entstehen Aldehyde.

Diese Kohlenstoff-Doppelbindungen können sowohl in internen Olefinen wie zum Beispiel in 2-Penten vorliegen, als auch in endständigen Olefinen wie zum Beispiel in 1,3-Butadien vorliegen. Zudem können die als Ausgangsstoffe eingesetzten Olefine entweder eine oder auch mehrere Kohlenstoff-Doppelbindungen enthalten.

Mit den erfindungsgemäßen Substanzen bzw. Katalysatoren, die entweder bereits als Metallkomplex vorgelegt oder *in situ* gebildet werden, können sowohl Olefine mit einer einfachen Kohlenstoff-Doppelbindung (zum Beispiel 2-Penten) als auch Olefine mit mehreren Kohlenstoff-Doppelbindungen (zum Beispiel 1,3-Butadien) hydroformyliert werden.

Mit den erfindungsgemäßen Substanzen bzw. Katalysatoren können insbesondere die in diesem Text als bevorzugt benannten Olefine effektiv zu Aldehyden umgesetzt werden.

Die vorliegende Erfindung betrifft zudem ein Verfahren zur Herstellung von Aldehyden umfassend die Verfahrensschritte
a) Herstellen oder Bereitstellen eines Reaktionssystems umfassend
   a-1) eine erste Komponente umfassend ein oder mehrere Olefine mit jeweils einer oder mehreren Kohlenstoff-Doppelbindungen,
   a-2) eine Katalysatorkomponente umfassend eine erfindungsgemäße Verbindung und eines oder mehrere Metallatome,
   a-3) eine zweite Komponente umfassend H₂ und CO
b) Erwärmen des Reaktionssystems, sodass Kohlenstoff-Doppelbindungen des einen oder mindestens eines der mehreren Olefine zu Aldehyden umgesetzt werden.

Aus Olefinen können über Hydroformylierungsreaktionen Aldehyde gewonnen werden. In einer Hydroformylierungsreaktion findet formal die Addition eines Wasserstoffs (-H) und einer Formylgruppe (-CHO) an eine Kohlenstoff-Doppelbindung eines Moleküls statt; somit entstehen Aldehyde. Hierzu muss in Schritt a) ein Reaktionssystem hergestellt oder bereitgestellt werden.

Eine erste Komponente des Reaktionssystems a-1) umfasst eines oder mehrere Olefine mit jeweils einer oder mehreren Kohlenstoff-Doppelbindungen. Diese Komponente umfasst somit die Ausgangsstoffe bzw. Edukte, welche zu Aldehyden umgesetzt werden. Die eingesetzten Olefine können sowohl interne Kohlenstoff-Dippelbindungen (zum Beispiel 2-Penten), als auch endständige Kohlenstoff-Doppelbindungen (zum Beispiel 1,3-Butadien) haben. Zudem können die Olefine eine oder mehrere Kohlenstoff-Doppelbindungen enthalten.

Eine weitere Komponente des Reaktionssystems a-2), die sogenannte Katalysatorkomponente, umfasst eine erfindungsgemäße Verbindung und eines oder mehrere Metallatome bzw. umfasst eine erfindungsgemäße Substanz. Die erfindungsgemäße Verbindung und das eine oder die mehreren Metallatome bilden den Katalysator der Hydroformylierungsreaktion. Werden die erfindungsgemäße Verbindung und das eine oder die mehreren Metallatome in Form eines Metallprecursors separat in das Reaktionssystem gegeben, so bildet sich der Katalysator *in situ.* Es können jedoch auch bereits isolierte Metallkomplexe direkt eingesetzt werden.

Eine zweite Komponente des Reaktionssystems a-3) umfasst H₂ und CO, auch Reaktivkomponente oder Synthesegas genannt. Die Herstellung von Aldehyden aus Olefinen wird formal durch Addition eines Wasserstoffs (-H) und einer Formylgruppe (-CHO) an eine Kohlenstoff-Doppelbindung gewährleistet. In der Praxis erfolgt die Umsetzung durch Zugabe von Wasserstoff (H₂) und Kohlenstoffmonoxid (CO) als Reaktivkomponente bzw. Synthesegas.

Durch das Erwärmen in Schritt b) findet die Reaktion statt und die Olefine werden zu den entsprechenden Aldehyden umgesetzt, wobei die Katalysatorkomponente die Reaktion beschleunigt.

Bevorzugt ist ein erfindungsgemäßes Verfahren, wobei das eine oder die mehreren Olefine ausgewählt ist/sind aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten.

Das erfindungsgemäße Verfahren eignet sich also zur Umsetzung einer Vielzahl an olefinischen Verbindungen.

Besonders bevorzugt ist ein erfindungsgemäßes Verfahren, wobei das eine oder die mehreren Olefine ausgewählt ist/sind der Gruppe bestehend aus cis- und/oder trans-2-Penten.

Das erfindungsgemäße Verfahren und/oder der Einsatz erfindungsgemäßer Verbindungen ist zudem geeignet zur Herstellung von linearen unverzweigten n-Aldehyden aus Olefinen mit endständigen und innenliegenden Doppelbindungen, sogenannte n-Selektivität.

Bevorzugt ist ein erfindungsgemäßes Verfahren (vorzugsweise wie vorstehend als bevorzugt bezeichnet), wobei das eine oder eines der mehreren Metallatome der Katalysatorkomponente ausgewählt ist aus der Gruppe bestehend aus Rhodium, Cobalt, Eisen, Ruthenium, Osmium, Iridium, Platin und Zinn, bevorzugt ist das eine oder eines der mehreren Metallatome Rhodium, besonders bevorzugt wird das Rhodium bereitgestellt aus einer der folgenden Verbindungen: Rh(acac)(CO)₂, [(acac)Rh(COD)] und Rh₄CO₁₂.

Bevorzugt ist das eine oder eines der mehreren Metallatome im Rahmen des Verfahrens der vorliegenden Erfindung Cobalt oder Rhodium. Cobalt und Rhodium haben sich als Metallatom in Katalysatoren für die Hydroformylierung als besonders geeignet erwiesen.

Das Rhodium wird innerhalb des erfindungsgemäßen Verfahrens bevorzugt aus den kommerziell erhältlichen und gut handhabbaren Verbindungen Rh(acac)(CO)₂, [(acac)Rh(COD)] und/oder Rh₄CO₁₂ bereitgestellt. Diese Verbindungen werden auch Metallprecusoren bezeichnet.

In einer ersten besonders bevorzugten Ausgestaltung der vorliegenden Erfindung wird das Rhodium bereitgestellt aus der Verbindung [(acac)Rh(COD)]; diese Verbindung entspricht (Acetylacetonat)(1,5-cyclooctadien)rhodium(I) mit der CAS-Nr. 12245-39-5.

In einer zweiten besonders bevorzugten Ausgestaltung der vorliegenden Erfindung wird das Rhodium bereitgestellt aus der Verbindung Rh(acac)(CO)₂; diese Verbindung entspricht (Acetylacetonat)dicarbonylrhodium(I) mit der CAS-Nr. 14874-82-9.

Bevorzugt ist ein erfindungsgemäßes Verfahren (vorzugsweise wie vorstehend als bevorzugt bezeichnet), wobei das molare Verhältnis von Metallatomen zu Verbindungen gemäß Formeln (I-i) bis (I-vii) in der Katalysatorkomponente im Bereich von 1:0,8 bis 1:4 liegt, bevorzugt im Bereich von 1:0,9 bis 1:2,1 liegt, besonders bevorzugt 1:1 oder 1:2 ist.

Die zuvor angegebenen bevorzugten molaren Verhältnisse von Metallatomen zu Verbindungen gemäß Formeln (I-i) bis (I-vii) erlauben hierbei eine besonders effiziente Nutzung der erfindungsgemäßen Verbindung, d.h. der Liganden, und Metallatome. Das molare Verhältnis sollte hierbei ausgeglichen sein. Ein hoher Überschuss an Liganden oder Metallatomen hingegen wäre in den meisten Fällen ineffizient, da die katalytische Aktivität der erfindungsgemäßen Verbindungen meist aus dem Zusammenwirken eines Liganden mit einem Metallatom oder zweier Liganden mit einem Metallatom oder eines Liganden mit zwei Metallatomen oder entsprechender Mischfälle beruht.

Bevorzugt ist ein erfindungsgemäßes Verfahren (vorzugsweise wie vorstehend als bevorzugt bezeichnet), wobei die Katalysatorkomponente ein Metallkomplex ist, oder einen Metallkomplex enthält, der Metallkomplex umfassend eine oder mehrere der erfindungsgemäßen Verbindungen.

Bevorzugt ist ein erfindungsgemäßes Verfahren (vorzugsweise wie vorstehend als bevorzugt bezeichnet), wobei die zweite Komponente umfassend H₂ und CO ein Gemisch aus H₂ und CO mit einem Stoffmengenverhältnis von H₂ zu CO im Bereich von 2:1 bis 1:2 ist, bevorzugt im Bereich von 1,5:1 bis 1:1,5, besonders bevorzugt im Bereich von 1,1:1 bis 1:1,1.

Formal entspricht diese Hydroformylierungsreaktion der Addition eines Wasserstoffs (-H) und einer Formylgruppe (-CHO) an eine Kohlenstoff-Doppelbindung. Hierfür werden in der Praxis in der Regel nahezu äquimolare Gemische von Wasserstoff (H₂) und Kohlenstoffmonoxid (CO) eingesetzt. Das besonders bevorzugte Stoffmengenverhältnis von H₂ zu CO liegt daher im Bereich von 1,1:1 bis 1:1,1, entspricht also einem ausgewogenen Verhältnis der Reaktivkomponenten.

Bevorzugt ist ein erfindungsgemäßes Verfahren (vorzugsweise wie vorstehend als bevorzugt bezeichnet), wobei das Reaktionssystem in Verfahrensschritt b) unter einem Druck im Bereich von 1 bis 30 bar steht, bevorzugt unter einem Druck im Bereich von 1,5 bar bis 25 bar steht.

Der bevorzugte Druckbereich des erfindungsgemäßen Verfahrens ist für Hydroformylierungsreaktionen vergleichsweise niedrig. Die Reaktion kann mithilfe der erfindungsgemäßen Verbindungen und/oder Substanzen, welche katalytisch wirken dennoch in vorteilhafter Weise effizient und mit hohen Umsätzen durchgeführt werden.

Bevorzugt ist ein erfindungsgemäßes Verfahren (vorzugsweise wie vorstehend als bevorzugt bezeichnet), wobei das Reaktionssystem in Verfahrensschritt b) auf eine Temperatur im Bereich von 80 °C bis 160 °C erwärmt wird, bevorzugt auf eine Temperatur im Bereich von 100 °C bis 140 °C erwärmt wird.

Die vorgenannten bevorzugten Temperaturbereiche des Reaktionssystems in Verfahrensschritt b) erlauben eine effiziente und zugleich vergleichsweise schonende Reaktionsführung bei hohen Umsätzen.

Bevorzugt ist ein erfindungsgemäßes Verfahren (vorzugsweise wie vorstehend als bevorzugt bezeichnet), wobei das Reaktionssystem zusätzlich ein Lösungsmittel umfasst, wobei das Lösungsmittel vorzugsweise Toluol umfasst oder Toluol ist.

Bevorzugt ist ein erfindungsgemäßes Verfahren (vorzugsweise wie vorstehend als bevorzugt bezeichnet), wobei der Stoffmengenanteil von Rhodium im Reaktionssystem im Bereich von 10 µmol/mol bis 1000 µmol/mol liegt, bevorzugt im Bereich von 20 µmol/mol bis 500 µmol/mol liegt, besonders bevorzugt im Bereich von 50 µmol/mol bis 200 µmol/mol liegt, jeweils bezogen auf die Stoffmenge an Olefinen im Reaktionssystem.

Bevorzugt ist ein erfindungsgemäßes Verfahren (vorzugsweise wie vorstehend als bevorzugt bezeichnet), wobei das Stoffmengenverhältnis von Rhodium zu den erfindungsgemäßen Verbindungen (vorzugsweise wie vorstehend als bevorzugt bezeichnet) in der Katalysatorkomponente im Bereich von 1:1 bis 1:3 liegt, bevorzugt im Bereich von 1:1,5 bis 1:2,5 liegt, besonders bevorzugt im Bereich von 1:1,9 bis 1:2,1 liegt.

Bevorzugt ist ein erfindungsgemäßes Verfahren (vorzugsweise wie vorstehend als bevorzugt bezeichnet), umfassend die Verfahrensschritte
V1) Vorlegen eines Olefins;
V2) Zugabe einer erfindungsgemäßen Verbindung und einer Substanz welche Rhodium umfasst;
V3) Zuführen von H₂ und CO;
V4) Erwärmen des Reaktionssystems aus V1) bis V3), wobei das Olefin zu einem Aldehyd umgesetzt wird.

Die bevorzugte Abfolge des erfindungsgemäßen Verfahrens gemäß Verfahrensschritten V1) bis V4) hat sich als besonders effektiv und effizient erwiesen. Die Erfindung ist jedoch nicht auf die zuvor genannte Reihenfolge der Verfahrensschritte beschränkt.

Die vorliegende Erfindung betrifft zudem die Verwendung einer erfindungsgemäßen Verbindung (vorzugsweise wie vorstehend als bevorzugt bezeichnet) als Ligand eines Katalysators oder einer erfindungsgemäßen Substanz (vorzugsweise wie vorstehend als bevorzugt bezeichnet) als Katalysator in einer Hydroformylierungsreaktion, bevorzugt in einer Hydroformylierungsreaktion gemäß einem erfindungsgemäßen Verfahren (vorzugsweise wie vorstehend als bevorzugt bezeichnet).

Durch Verwendung der erfindungsgemäßen Verbindungen als Ligand eines Katalysators konnte in Hydroformylierungsreaktionen ein hoher Umsatz erreicht werden. Des Weiteren zeigen die mit diesen Liganden hergestellten Katalysatoren die Eigenschaft, interne Olefine (zum Beispiel 2-Penten) in einem nicht unerheblichen Teil zu terminalen Aldehyden zu hydroformylieren, sogenannte n-Selektivität.

Auch die erfindungsgemäßen Substanzen können als Katalysator in einer Hydroformylierungsreaktion verwendet werden. Der Katalysator ist ein Metallkomplex wie vorstehend definiert. Dieser Metallkomplex wird bevorzugt *in situ* durch Zusammengeben der erfindungsgemäßen Verbindung, d.h. des Liganden, und des Metallprecursors erzeugt.

Der Metallkomplex kann jedoch auch separat hergestellt, isoliert und als Katalysator in einer Hydroformylierungsreaktion eingesetzt werden oder als Katalysator für eine Hydroformylierungsreaktion an Dritte verkauft werden.

### BEISPIELE

Die folgenden Beispiele dienen der weiteren Erläuterung und Veranschaulichung der vorliegenden Erfindung, ohne deren Anwendungsbereich einzuschränken.

### 1) Synthese von erfindungsgemäßen Verbindungen:

### Verbindung I-i:

### 6,6'-((4,4'-Dibrom-[2,2'-binaphthalen]-1,1'-diyl)bis(oxy))bis(4,8-di-tert-butyl-2,10-dimethoxydi-benzo[d,f][1,3,2]dioxaphosphepin)

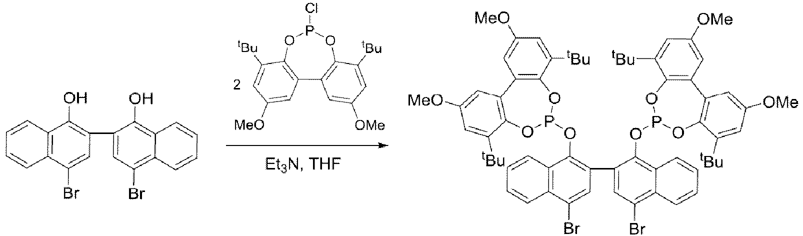

Zu einer Lösung von 4,4'-Dibrom-[2,2'-binaphthalen]-1,1'-diol (339 mg, 0,76 mmol) und Triethylamin (232 mg, 2,29 mmol) in THF (4 ml) wird unter Rühren und bei 0°C das 4,8-Di-*tert*-butyl-6-chlor-2,10-dime-thoxydibenzo[*d,f*][1,3,2]dioxaphosphepin (678 mg, 1,60 mmol) in THF (6 ml) langsam zugetropft und anschließend bei Raumtemperatur über Nacht weitergerührt. Zur Aufarbeitung wird vom ausgefallenem Hydrochlorid abfiltriert und das Lösungsmittel unter Vakuum entfernt. Der erhaltene rot-braune Feststoff wird bei 60°C getrocknet. Zur Reinigung wurde der Rückstand mit Acetonitril umkristallisiert, wobei sich aus der Mutterlauge durch Verringerung des Volumens weiteres Produkt gewinnen lässt.

Ausbeute an Verbindung **I-i:** 555 mg hellbrauner Feststoff (59%).

³¹P-NMR (CD₂Cl₂): □ +136,6 ppm.

¹H-NMR (CD₂Cl₂):□□ 8,22 (2H, br d, *J* 8,2 Hz, 2xHₐᵣ), 8,14 (2H, d, *J* 8,4 Hz, 2xHₐᵣ), 7,70 (2H, br s, 2xHₐᵣ), 7,64 (2H, ddd, *J* 8,4, 6,9, 1.3 Hz, 2xHₐᵣ), 7,51 (2H, m, 2xHₐᵣ), 6,90 (2H, br s, 2xHₐᵣ), 6,67 (4H, br s, 4xHₐᵣ), 6,52 (2H, br s, 2xHₐᵣ), 3,79 (12H, s, 4xOCH₃), 1,10 (18H, br s, 2xC(CH₃)₃), 0,88 (18H, br s, 2xC(CH₃)₃).

HRMS (ESI-TOF) [M+H]⁺: *m*/*z* berechnet: für C₆₄H₆₆⁷⁹Br₂O₁₀P₂ 1215,2576, gefunden: 1215,2596.

HRMS (ESI-TOF) [M+Na]⁺: *m*/*z* berechnet: für C₆₄H₆₆⁷⁹Br₂O₁₀P₂ 1237,2390, gefunden: 1237,2419.
Elementaranalyse: berechnet: C 63,17% H 5,47% P 5,09% gefunden: C 63,15% H 5,41% P 4,97%.

### Verbindung I-ii:

### (R,R)-4,4'-((4,4'-Dibrom-[2,2'-binaphthalen]-1,1'-diyl)bis(oxy))didinaphtho[2,1-d:1',2'-f][1,3,2]dioxaphos-phepin

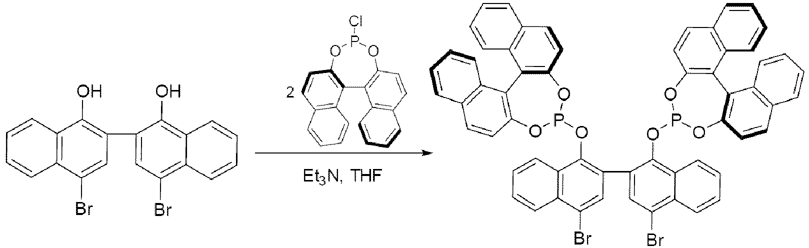

Zu einer Lösung von 4,4'-Dibrom-[2,2'-binaphthalen]-1,1'-diol (264 mg, 0,59 mmol) und Triethylamin (180 mg, 1,78 mmol) in THF (3 ml) wird unter Rühren und bei 0°C das (*R*)-4-chlordinaphtho[2,1-*d*:1',2'-*f*][1,3,2]dioxaphosphepin (438 mg, 1,25 mmol) in THF (5 ml) langsam zugetropft und anschließend bei Raumtemperatur über Nacht weitergerührt. Zur Aufarbeitung wird vom ausgefallenem Hydrochlorid abfiltriert und das Lösungsmittel unter Vakuum entfernt. Der erhaltene Feststoff wird bei 60°C getrocknet. Die Reinigung erfolgte durch Säulenchromatographie (Eluent: Heptan/Dichlormethan 1/2). Durch axiale Chiralität im Rückgrat liegen zwei Diastereomere vor.

Ausbeute an Verbindung **I-ii**: 445 mg hell-gelber Feststoff (70%).

³¹P-NMR (CD₂Cl₂): □ +154,2 ppm (d, *J* 13,6 Hz, Diastereomer A) und +144,1 ppm (d, *J* 13,6 Hz, Diastereomer B).

HRMS (ESI-TOF) [M+H]⁺: *m*/*z* berechnet: für C₆₀H₃₄⁷⁹Br₂O₆P₂ 1071,0276, gefunden 1071,0287.

HRMS (ESI-TOF) [M+Na]⁺: *m*/*z* berechnet: für C₆₀H₃₄⁷⁹Br₂O₆P₂ 1093,0090, gefunden 1093,0112.
Elementaranalyse: berechnet: C 67,18% H 3,19% P 5,78% gefunden: C 67,22% H 3,28% P 5,67%.

### Verbindung I-iii:

### 2,2'-((4,4'-Dibrom-[2,2'-binaphthalen]-1,1'-diyl)bis(oxy))bis(4,5-diphenyl-1,3,2-dioxaphosphol)

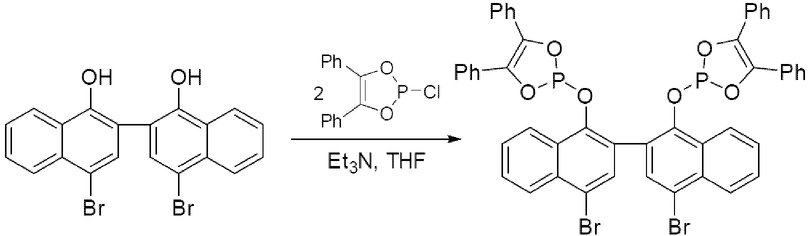

Zu einer Lösung von 4,4'-Dibrom-[2,2'-binaphthalen]-1,1'-diol (300 mg, 0,68 mmol) und Triethylamin (205 mg, 2,03 mmol) in THF (3 ml) wird unter Rühren und bei 0°C das 2-Chlor-4,5-diphenyl-1,3,2-dio-xaphosphol (392 mg, 1,42 mmol) in THF (3 ml) langsam zugetropft und anschließend bei Raumtemperatur über Nacht weitergerührt. Zur Aufarbeitung wird vom ausgefallenem Hydrochlorid abfiltriert und das Lösungsmittel unter Vakuum entfernt. Zur Reinigung wurde der feste Rückstand aus Acetonitril umkristallisiert.

### Ausbeute an Verbindung I-iii: 360 mg hell-gelber Feststoff (57%).

³¹P-NMR (CD₂Cl₂): □ +126,2 ppm.

¹H-NMR (CD₂Cl₂):□□ 8,29 (2H, m, 2xHₐᵣ), 8,23 (2H, m, 2xHₐᵣ), 7,73-7,58 (4H, m, 4xHₐᵣ), 7,55 (2H, s, 2xHₐᵣ), 7,31-7,05 (20H, m, 20xHₐᵣ).

¹³C-NMR (CD₂Cl₂): □ 145,6 (2xCₐᵣ), 135,2 (m, 4xO-C=), 133,1 (2xCₐᵣ), 132,6 (2xCₐᵣH), 129,6 (m, 2xCₐᵣ), 129,4 (4xCₐᵣ), 128,9 (4xCₐᵣH), 128,7 (10xCₐᵣH), 127,9 (2xCₐᵣH), 127,6 (2xCₐᵣH), 127,5 (8xCₐᵣH), 126,4 (m, 2xCₐᵣ), 124,3 (2xCₐᵣH), 118,2 (2xCₐᵣ-Br).

HRMS (ESI-TOF) [M+H]⁺: *m*/*z* berechnet: für C₄₈H₃₀⁷⁹Br₂O₆P₂ 922,9963, gefunden: 922,9946.

HRMS (ESI-TOF) [M+Na]⁺: *m*/*z* berechnet: für C₄₈H₃₀⁷⁹Br₂O₆P₂ 944,9777, gefunden: 944,9764.
Elementaranalyse: berechnet: C 62,36% H 3,27% P 6,70% gefunden: C 62,53% H 3,44% P 6,64%.

### Verbindung I-iv:

### 2,2'-((4,4'-Dibrom-[2,2'-binaphthalen]-1,1'-diyl)bis(oxy))bis(5,5-dimethyl-1,3,2-di-oxaphosphinan)

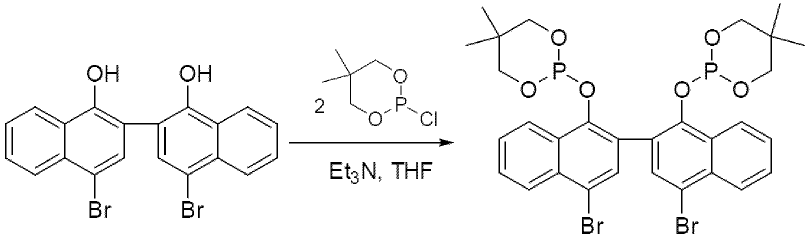

Zu einer Lösung von 4,4'-Dibrom-[2,2'-binaphthalen]-1,1'-diol (429 mg, 0,97 mmol) und Triethylamin (293 mg, 2,90 mmol) in THF (3 ml) wird unter Rühren und bei 0°C das 2-Chlor-5,5-dimethyl-1,3,2-dio-xaphosphinan (342 mg, 2,03 mmol) in THF (3 ml) langsam zugetropft und anschließend bei Raumtemperatur über Nacht weitergerührt. Zur Aufarbeitung wird vom ausgefallenem Hydrochlorid abfiltriert und das Lösungsmittel unter Vakuum entfernt. Zur Reinigung wurde der feste Rückstand aus Acetonitril umkristallisiert.

### Ausbeute an Verbindung I-iv: 595 mg hell-brauner Feststoff (87%).

³¹P-NMR (CD₂Cl₂): □ +119,0 ppm.

¹H-NMR (CD₂Cl₂): □ 8,30-8,23 (4H, m, 4xHₐᵣ), 7,93 (2H, s, 2xHₐᵣ), 7,75-7,62 (4H, m, 4xHₐᵣ), 4,06 (4H, m, 2xHₐ-CH₂O), 3,14 (4H, m, 2xH_{b}-CH₂O), 1,10 (6H, s, 2xCH₃), 0,61 (6H, s, 2xCH₃).

¹³C-NMR (CD₂Cl₂): □ 147,6 (m. 2xCₐᵣ-O), 133,1 (2xCₐᵣH), 133,0 (2xCₐᵣ), 130,2 (2xCₐᵣ), 128,5 (2xCₐᵣH), 127,7 (2xCₐᵣH), 127,6 (2xCₐᵣH), 127,2 (m, Cₐᵣ), 124,0 (m, 2xCₐᵣH), 117,0 (Cₐᵣ-Br), 70,1 (d, *J* 11.4 Hz, 4xCH₂O), 32,7 (m, 2x^{t}C), 22,6 (2xCH₃), 22,2 (2xCH₃).

HRMS (ESI-TOF) [M+H]⁺: *m*/*z* berechnet: für C₃₀H₃₀⁷⁹Br₂O₆P₂ 706,9963, gefunden: 706,9960

HRMS (ESI-TOF) [M+Na]⁺: *m*/*z* berechnet: für C₃₀H₃₀⁷⁹Br₂O₆P₂ 728,9777, gefunden: 728,9786.
Elementaranalyse: berechnet: C 50,87% H 4,27% P 8,75% gefunden: C 50,76% H 4,43% P 8,70%.

### Verbindung I-v:

### 8,11-Dibrom-2-((3,3'-di-tert-butyl-5,5'-dimethoxy-2'-((4,4,5,5-tetraphenyl-1,3,2-dioxaphospholan-2-yl)oxy)-[1,1'-biphenyl]-2-yl)oxy)dinaphtho[1,2-d:2',1'-f][1,3,2]dioxaphosphepin

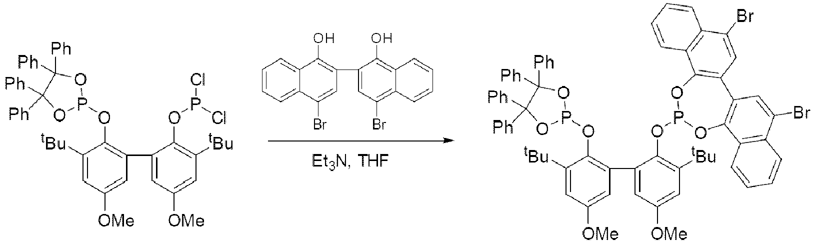

Zu einer Lösung von 2-((3,3'-Di-*tert*-butyl-2'-((dichlorphosphanyl)oxy)-5,5'-dimethoxy-[1,1'-biphenyl]-2-yl)oxy)-4,4,5,5-tetraphenyl-1,3,2-dioxaphospholan (492 mg, 0,58 mmol) in THF (5 ml) wird unter Rühren und bei 0°C eine Lösung von 4,4'-Dibrom-[2,2'-binaphthalen]-1,1'-diol (256 mg, 0,58 mmol) und Triethylamin (175 mg, 1,73 mmol) in THF (3 ml) tropfenweise hinzugegeben. Zur Vervollständigung der Reaktion wird anschließend bei Raumtemperatur über Nacht weitergerührt. Zur Aufarbeitung wird vom ausgefallenem Hydrochlorid abfiltriert und das Lösungsmittel unter Vakuum entfernt. Zur Reinigung wurde der feste Rückstand mit Acetonitril in der Hitze suspendiert und nach dem Abkühlen abfiltriert.

### Ausbeute an Verbindung I-v: 620 mg hell-brauner Feststoff (88%).

³¹P-NMR (CD₂Cl₂): □ +149,5 ppm (d, *J* 118 Hz) und +142,9 ppm (d, *J* 118 Hz).

¹H-NMR (CD₂Cl₂): □ 8,34-8,18 (4H, m, 4xHₐᵣ), 8,01 (2H, d, *J* 12.4 Hz, 2xHₐᵣ), 7,72-7,53 (5H, m, 5xHₐᵣ),7,37-6,90 (22H, m, 22xHₐᵣ), 6,68 (1H, d, *J* 3.1 Hz, Hₐᵣ), 3,86 (3H, s, OCH₃), 3,56 (3H, s, OCH₃), 1,16 (9H, s, C(CH₃)₃), 0,96 (9H, s, C(CH₃)₃).

HRMS (ESI-TOF) [M+Na]⁺: *m*/*z* berechnet: für C₆₈H₅₈⁷⁹Br₂O₈P₂ 1245,1866, gefunden: 1245,1841.
Elementaranalyse: berechnet: C 66,68% H 4,77% P 5,06% gefunden: C 66,56% H 4,84% P 5,12%.

### Verbindung I-vi:

### 8,11-Dibrom-2-((3,3'-di-tert-butyl-2'-((4,8-di-tert-butyl-2,10-dimethoxydibenzo[d,f][1,3,2]dioxaphosphepin-6-yl)oxy)-5,5'-dimethoxy-[1,1'-biphenyl]-2-yl)oxy)dinaphtho[1,2-d:2',1'-f][1,3,2]dioxaphosphepin

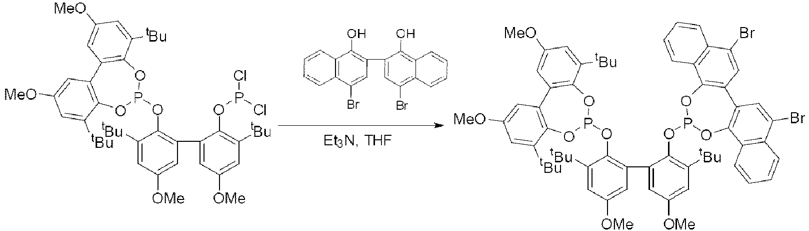

Zu einer Lösung von 4,8-Di*-tert*-butyl-6-((3,3'-di-tert-butyl-2'-((dichlorophosphanyl)oxy)-5,5'-dimethoxy-[1,1'-biphenyl]-2-yl)oxy)-2,10-dimethoxydibenzo[*d*,*f*][1,3,2]dioxaphosphepin (513 mg, 0,61 mmol) in THF (5 ml) wird unter Rühren und bei 0°C eine Lösung von 4,4'-Dibrom-[2,2'-binaphthalen]-1,1'-diol (269 mg, 0,61 mmol) und Triethylamin (184 mg, 1,82 mmol) in THF (3 ml) tropfenweise hinzugegeben. Zur Vervollständigung der Reaktion wird anschließend bei Raumtemperatur über Nacht weitergerührt. Zur Aufarbeitung wird vom ausgefallenem Hydrochlorid abfiltriert und das Lösungsmittel unter Vakuum entfernt. Zur Reinigung wurde der feste Rückstand mit heißem Acetonitril suspendiert und nach dem Abkühlen abfiltriert.

### Ausbeute an Verbindung I-vi: 365 mg hell-brauner Feststoff (49%).

³¹P-NMR (CD₂Cl₂): □ +147,0 ppm (d, *J* 1,6 Hz) und +141,3 ppm (d, *J* 1,6 Hz).

¹H-NMR (CD₂Cl₂): □ 8,25 (2H, m, 2xHₐᵣ), 8,15 (1H, br d, *J* 8,4 Hz, Hₐᵣ), 7,90 (2H, d, *J* 5,6 Hz, 2xHₐᵣ), 7,72 (1H, ddd, *J* 8,4, 6,9, 1,3 Hz, Hₐᵣ), 7,65 (1H, ddd, *J* 8,3, 6,9, 1,2 Hz, Hₐᵣ), 7,59 (1H, ddd, *J* 8,3, 6,9, 1,2 Hz, Hₐᵣ), 7,50 (1H, br d, *J* 8,3 Hz, Hₐᵣ), 7,28 (1H, ddd, *J* 8,3, 6,9, 1,2 Hz, Hₐᵣ), 7,07 (2H, dd, *J* 2,9, 2,9 Hz, 2xHₐᵣ), 6,94 (1H, d, *J* 3,2 Hz, Hₐᵣ), 6,91 (1H, d, *J* 3.1 Hz, Hₐᵣ), 6,87 (1H, d, *J* 3,2 Hz, Hₐᵣ), 6,81 (1H, d, *J* 3,2 Hz, Hₐᵣ), 6,70 (1H, d, *J* 3,1 Hz, Hₐᵣ), 6,68 (1H, d, *J* 3,1 Hz, Hₐᵣ), 3,86 (3H, s, OCH₃), 3,84 (3H, s, OCH₃), 3,77 (3H, s, OCH₃), 3,70 (3H, s, OCH₃), 1,56 (9H, s, C(CH₃)₃), 1,25 (9H, s, C(CH₃)₃), 1,07 (9H, s, C(CH₃)₃), 0,92 (9H, s, C(CH₃)₃).

¹³C-NMR (CD₂Cl₂): □ 156,3 (Cₐᵣ-OMe), 156,0 (Cₐᵣ-OMe), 156,0 (Cₐᵣ-OMe), 155,8 (Cₐᵣ-OMe), 147,4 (d, *J* 9,9 Hz, Cₐᵣ), 144,8 (Cₐᵣ), 144,6 (d, J 2,4 Hz, Cₐᵣ), 144,4 (m, Cₐᵣ), 144,2 (m, Cₐᵣ), 143,5 (d, J 8,7 Hz, Cₐᵣ), 143,4 (Cₐᵣ), 143,0 (d, *J* 2,0 Hz, Cₐᵣ), 142,6 (Cₐᵣ), 141,0 (d, *J* 3,7 Hz, Cₐᵣ), 134,6 (d, *J* 4,7 Hz, Cₐᵣ), 133,7 (d, *J* 3,5 Hz, Cₐᵣ), 132,7 (Cₐᵣ), 132,5 (Cₐᵣ), 132,5 (m, Cₐᵣ), 132,1 (m, Cₐᵣ), 130,7 (CₐᵣH), 130,5 (CₐᵣH), 129,4 (Cₐᵣ), 129,1 (Cₐᵣ), 128,8 (CₐᵣH), 128,5 (CₐᵣH), 127,8 (CₐᵣH), 127,5 (CₐᵣH), 127,3 (CₐᵣH), 127,2 (Cₐᵣ), 127,2 (CₐᵣH), 124,9 (d, *J* 3,3 Hz, Cₐᵣ), 123,5 (CₐᵣH), 123,1 (CₐᵣH), 119,4 (Cₐᵣ), 118,2 (Cₐᵣ), 115,7 (CₐᵣH), 115,3 (CₐᵣH), 115,1 (d, *J* 3,3 Hz, CₐᵣH), 114,9 (CₐᵣH), 114,8 (CₐᵣH), 114,5 (CₐᵣH), 113,3 (2xCₐᵣH), 56,1 (OCH₃), 56,0 (2xOCH₃), 55,9 (OCH₃), 35,8 (^{t}C), 35,8 (^{t}C), 35,5 (^{t}C), 35,5 (^{t}C), 31,5 (3xCH₃), 31,3 (3xCH₃), 31,1 (d, *J* 4,2 Hz, 3xCH₃), 30,3 (3xCH₃).

HRMS (ESI-TOF) [M+H]⁺: *m*/*z* berechnet: für C₆₄H₆₆⁷⁹Br₂O₁₀P₂ 1215,2576, gefunden: 1215,2595.

HRMS (ESI-TOF) [M+Na]⁺: *m*/*z* berechnet: für C₆₄H₆₆⁷⁹Br₂O₁₀P₂ 1237,2390, gefunden: 1237,2417.
Elementaranalyse: berechnet: C 63,17% H 5,47% P 5,09% gefunden: C 62,99% H 5,56% P 5,11%.

### Verbindung I-vii:

### 6,6'-((4,4'-Dibromo-[2,2'-binaphthalen]-1,1'-diyl)bis(oxy))bis(2,4,8,10-tetra-tert-butyldibenzo[d,f][1,3,2]dio-xaphosphepin)

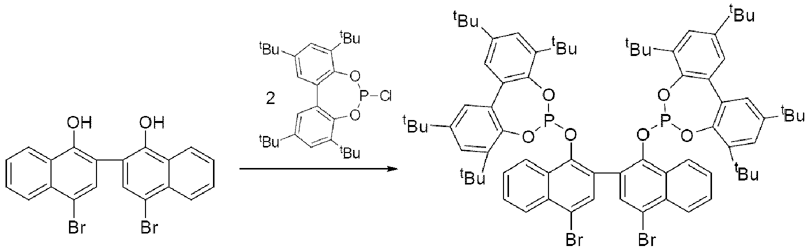

Zu einer Lösung von 4,4'-Dibrom-[2,2'-binaphthalen]-1,1'-diol (222 mg, 0,50 mmol) und Triethylamin (152 mg, 1,50 mmol) in Toluol (3 ml) wird bei 0°C eine Lösung von 2,4,8,10-Tetra-*tert*-butyl-6-chlorodi-benzo[*d*,*f*][1,3,2]dioxaphosphepin (523 mg, 1,10 mmol) in Toluol (2 ml) unter Rühren hinzugegeben. Zur Vervollständigung der Reaktion wird anschließend drei Tage bei Raumtemperatur weitergerührt. Zur Aufarbeitung wird vom ausgefallenem Hydrochlorid abfiltriert und das Lösungsmittel unter Vakuum entfernt. Zur Reinigung wurde der feste Rückstand zunächst durch Säulenchromatographie vorgereinigt (Eluent: Cyclohexan/AcOEt 99/1) und anschließend aus wenig *n*-Hexan umkristallisiert.

### Ausbeute an Verbindung I-vii: 110 mg hell-brauner Feststoff (16%).

Schmelztemperatur: 292-293°C.

³¹P-NMR (CD₂Cl₂): □ +137,5 ppm.

¹H-NMR (CD₂Cl₂): □ 8,19 (2H, dm, *J* 8,4 Hz, 2xHₐᵣ), 8,08 (2H, br d, *J* 8,4 Hz, 2xHₐᵣ), 7,94 (2H, m, 2xHₐᵣ), 7,61 (2H, ddd, 8,4, 6,9, 1,3 Hz, 2xHₐᵣ), 7,38 (2H, m, 2xHₐᵣ), 7,38 (4H, d, *J* 2,5 Hz, 4xHₐᵣ), 7,19 (4H, d, *J* 2,5 Hz, 4xHₐᵣ), 1,36 (36H, s, 4xC(CH₃)₃), 1.15 (18H, br s, 2xC(CH₃)₃), 0.93 (18H, br s, 2xC(CH₃)₃).

¹³C-NMR (CD₂Cl₂): □ 147,5 (m, 2xCₐᵣ), 147,5 (m, 2xCₐᵣ), 145,8 (2xCₐᵣ), 141,0 (2xCₐᵣ), 133,3 (2xCₐᵣH), 133,1 (m, 2xCₐᵣ), 130,5 (2xCₐᵣ), 128,6 (2xCₐᵣH), 127,4 (2xCₐᵣH), 127,1 (4xCₐᵣH), 126,4 (m, 2xCₐᵣ), 125,7 (2xCₐᵣH), 124,8 (4xCₐᵣH), 117,9 (2xCₐᵣ-Br), 35,4 (4x^{t}c), 34,9 (4x^{t}C), 31,7 (12xCH₃), 30,7 (12xCH₃).

HRMS (ESI-TOF) [M+H]⁺: *m*/*z* berechnet: für C₇₆H₉₀⁷⁹Br⁸¹BrO₆P₂ 1321,4633, gefunden: 1321,4741.

HRMS (ESI-TOF) [M+Na]⁺: *m*/*z* berechnet: für C₇₆H₉₀⁷⁹Br⁸¹BrO₆P₂ 1343,4452, gefunden: 1343,4568.

### 2) Einsatz erfindungsgemäßer Verbindungen und Substanzen in Hydroformylierungsreaktionen

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung des Einflusses von Feuchtigkeit und Sauerstoff wurde das als Solvens benutzte Toluol in einem Pure Solv. MD-7 System gereinigt und unter Argon aufbewahrt. Cis/trans-2-Penten (Aldrich) wurde über Natrium am Rückfluss erhitzt und unter Argon destilliert. Im Autoklav wurden unter Argonatmosphäre Lösungen der Katalysatorvorstufe (Metallprecursor) und der erfindungsgemäßen Verbindungen als Liganden jeweils in Toluol gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur wurde das in der Druckpipette befindliche Olefin mit einem Überdruck von ca. 3 bar in den Autoklaven gepresst. Die Reaktion wurde bei konstantem Druck (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischung wurden entnommen, mit 10 ml Pentan verdünnt und mittels Gaschromatographie analysiert: HP 5890 Series II plus, PONA, 50 m x 0,2 mm x 0,5 µm.

Die in Tabelle 1 dargestellten Ergebnisse der Hydroformylierung wurden unter folgenden Bedingungen / Parametern erhalten:
- eingesetztes Olefin: 2-Penten (2,41 M),
- Temperatur: 120°C,
- Reaktionszeit: 4 h,
- Druck: 20 bar,
- Zusammensetzung des Synthesegases: CO/H₂ (1:1),
- Lösungsmittel: Toluol,
- eingesetzte Menge an Rhodium in Form von [(acac)Rh(COD)] bezogen auf die eingesetzte Menge an 2-Penten: µmol/mol,
- molares Verhältnis von Rhodiumatomen zu eingesetzter Verbindung 1:2.

**Tabelle 1. Ergebnisse in der Hydroformylierung von 2-Penten**

| Verbindung | Umsatz (%) | n-Selektivität (%) |
|---|---|---|
| I-i | 29 | 73,4 |
| I-ii | 89 | 81,6 |
| I-iii | 10 | 58,9 |
| I-iv | 81 | 54,6 |
| I-v | 59 | 74,9 |
| I-vi | 75 | 73,4 |
| I-vii | 15 | 74,5 |

Anhand der in Tabelle 1 dargestellten Ergebnisse konnte gezeigt werden, dass die erfindungsgemäßen Verbindungen I-i bis I-vii und entsprechende Substanzen sowie auch das erfindungsgemäße Verfahren in der Hydroformylierung von Olefinen, hier beispielhaft gezeigt an dem Substrat 2-Penten, hervorragende Resultate zeigen.

Die eingesetzten erfindungsgemäßen Verbindungen I-i bis I-vii zeigten gute Umsätze bei der Hydroformylierung von 2-Penten in die entsprechenden Aldehyde.

Im Rahmen der vorliegenden Erfindung ist es somit gelungen durch gezielte Wahl von Organobisphosphit-Verbindungen mit den in den Ansprüchen und der Beschreibung definierten Strukturformeln bisher unbekannte Organphosphit-Liganden als Basis für Katalysatoren in der Hydroformylierung bereitzustellen, welche vorteilhaft hohe Umsätze erzielen.

Hierbei zeigte sich auch, dass die katalytische Aktivität der eingesetzten Verbindungen in der Hydroformylierung insbesondere in Kombination mit Rhodium-Metallzentren besonders effektiv ist.

Des Weiteren zeigen die Ergebnisse, dass die erfindungsgemäßen Verbindungen I-i bis I-vii ebenfalls dazu in der Lage sind, gute bis exzellente Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier untersuchten 2-Penten der Fall, eine besonders schwer einzustellende Größe ist. Folglich sind die mit den erfindungsgemäßen Verbindungen I-i bis I-vii erzielten n-Selektivitäten, d.h. die Selektivität für das Produkt Hexanal, von bis zu 81,6% sehr gute Resultate.

## Patentansprüche

1. Verbindung, wobei die Verbindung ein Organobisphosphit ist, ausgewählt aus den folgenden Formeln (I-i) bis (I-vii) 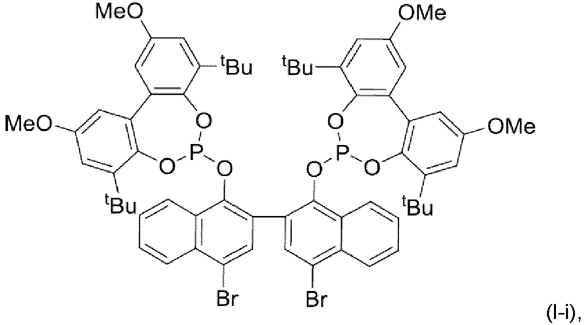 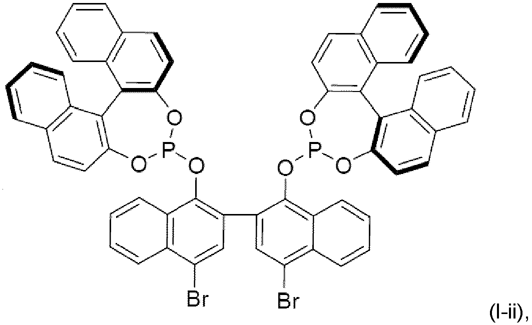 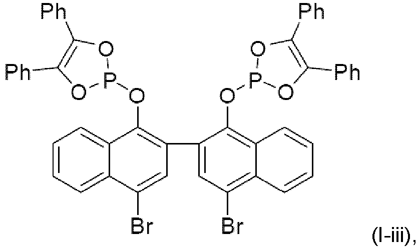 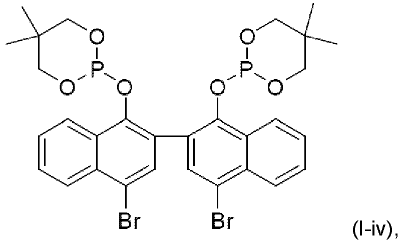 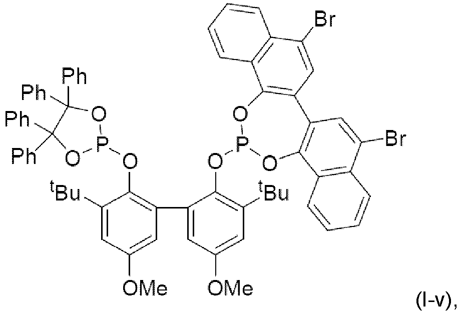 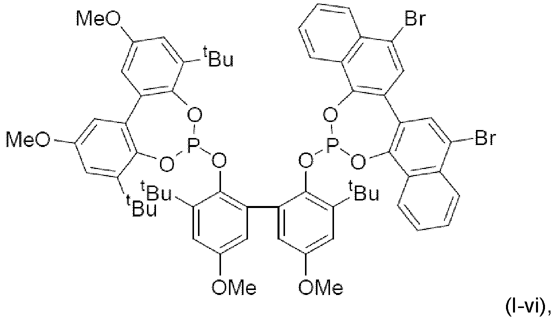 oder

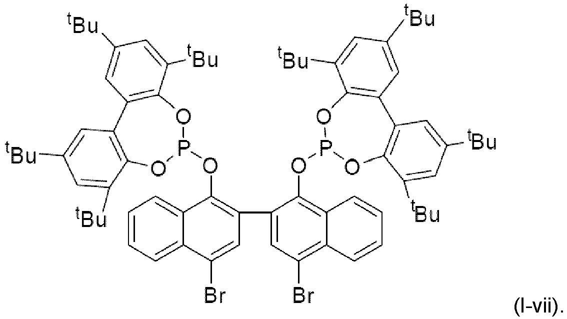

2. Substanz, wobei die Substanz
i) eine Mischung ist, die Mischung umfassend
- eine oder mehrere der Verbindungen ausgewählt aus den Formeln (I-i) bis (I-vii) nach Anspruch 1,
und
- eines oder mehrere Metallatome,
und/oder
ii) ein Metallkomplex ist oder einen Metallkomplex enthält, der Metallkomplex umfassend
- eine oder mehrere der Verbindungen ausgewählt aus den Formeln (I-i) bis (I-vii) nach Anspruch 1,
und
- eines oder mehrere Metallatome.

3. Substanz nach Anspruch 2, wobei
- das molare Verhältnis von Metallatomen zu Verbindungen gemäß Formeln (I-i) bis (I-vii) in der Mischung und/oder im Metallkomplex im Bereich von 0,5:1 bis 1:4 liegt,
bevorzugt im Bereich von 0,9:1 bis 1:2,1 liegt, besonders bevorzugt 1:1 oder 1:2 ist,
und/oder
- das ein oder eines der mehreren Metallatome in der Mischung und/oder im Metallkomplex ausgewählt ist aus der Gruppe bestehend aus Rhodium, Cobalt, Eisen, Ruthenium, Osmium, Iridium, Platin und Zinn,
bevorzugt ist das ein oder eines der mehreren Metallatome Rhodium,
und/oder
- die Mischung als weiteren Bestandteil umfasst
- ein oder mehrere Olefine mit jeweils einer oder mehreren Kohlenstoff-Doppelbindungen,
bevorzugt ausgewählt aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
und/oder
- Lösungsmittel, wobei das Lösungsmittel vorzugsweise Toluol umfasst oder Toluol ist.

4. Verfahren zur Herstellung von Aldehyden umfassend die Verfahrensschritte
a) Herstellen oder Bereitstellen eines Reaktionssystems umfassend
a-1) eine erste Komponente umfassend ein oder mehrere Olefine mit jeweils einer oder mehreren Kohlenstoff-Doppelbindungen,
a-2) eine Katalysatorkomponente umfassend eine Verbindung gemäß Anspruch 1 und eines oder mehrere Metallatome,
a-3) eine zweite Komponente umfassend H₂ und CO
b) Erwärmen des Reaktionssystems, sodass Kohlenstoff-Doppelbindungen des einen oder mindestens eines der mehreren Olefine zu Aldehyden umgesetzt werden.

5. Verfahren nach Anspruch 4, wobei das eine oder die mehreren Olefine ausgewählt ist/sind aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
bevorzugt ausgewählt aus der Gruppe bestehend aus cis- und/oder trans-2-Penten.

6. Verfahren nach einem der Ansprüche 4 oder 5, wobei
- das eine oder eines der mehreren Metallatome der Katalysatorkomponente ausgewählt ist aus der Gruppe bestehend aus Rhodium, Cobalt, Eisen, Ruthenium, Osmium, Iridium, Platin und Zinn,
bevorzugt ist das eine oder eines der mehreren Metallatome Rhodium,
besonders bevorzugt wird das Rhodium bereitgestellt aus einer der folgenden Verbindungen: Rh(acac)(CO)₂, [(acac)Rh(COD)] und Rh₄CO₁₂,
und/oder
- das molare Verhältnis von Metallatomen zu Verbindungen gemäß Formeln (I-i) bis (I-vii) in der Katalysatorkomponente im Bereich von 1:0,8 bis 1:4 liegt,
bevorzugt im Bereich von 1:0,9 bis 1:2,1 liegt,
besonders bevorzugt 1:1 oder 1:2 ist,
und/oder
- die Katalysatorkomponente ein Metallkomplex ist oder einen Metallkomplex enthält, der Metallkomplex umfassend eine oder mehrere der Verbindungen nach Anspruch 1 und Rhodium.

7. Verfahren nach einem der Ansprüche 4 bis 6, wobei die zweite Komponente umfassend H₂ und CO ein Gemisch aus H₂ und CO mit einem Stoffmengenverhältnis von H₂ zu CO im Bereich von 2:1 bis 1:2 ist, bevorzugt im Bereich von 1,5:1 bis 1:1,5, besonders bevorzugt im Bereich von 1,1:1 bis 1:1,1.

8. Verfahren nach einem der Ansprüche 4 bis 7, wobei das Reaktionssystem in Verfahrensschritt b)
- unter einem Druck im Bereich von 1 bis 30 bar steht, bevorzugt unter einem Druck im Bereich von 1,5 bar bis 25 bar steht,
und/oder
- auf eine Temperatur im Bereich von 80 °C bis 160 °C erwärmt wird, bevorzugt auf eine Temperatur im Bereich von 100 °C bis 140 °C erwärmt wird.

9. Verfahren nach einem der Ansprüche 4 bis 8, wobei
- das Reaktionssystem zusätzlich ein Lösungsmittel umfasst, wobei das Lösungsmittel vorzugsweise Toluol umfasst oder Toluol ist,
und/oder
- der Stoffmengenanteil von Rhodium im Reaktionssystem im Bereich von 10 µmol/mol bis 1000 µmol/mol liegt, bevorzugt im Bereich von 20 µmol/mol bis 500 µmol/mol liegt, besonders bevorzugt im Bereich von 50 µmol/mol bis 200 µmol/mol liegt, jeweils bezogen auf die Stoffmenge an Olefinen im Reaktionssystem,
und/oder
- wobei das Stoffmengenverhältnis von Rhodium zu den Verbindungen gemäß Anspruch 1 in der Katalysatorkomponente im Bereich von 1:1 bis 1:3 liegt, bevorzugt im Bereich von 1:1,5 bis 1:2,5 liegt, besonders bevorzugt im Bereich von 1:1,9 bis 1:2,1 liegt.

10. Verfahren nach einem der Ansprüche 4 bis 9, umfassend die Verfahrensschritte
V1) Vorlegen eines Olefins;
V2) Zugabe einer Verbindung gemäß Anspruch 1 und einer Substanz welche Rhodium umfasst;
V3) Zuführen von H₂ und CO;
V4) Erwärmen des Reaktionssystems aus V1) bis V3), wobei das Olefin zu einem Aldehyd umgesetzt wird.

11. Verwendung einer Verbindung nach Anspruch 1 als Ligand eines Katalysators oder einer Substanz nach einem der Ansprüche 2 oder 3 als Katalysator in einer Hydroformylierungsreaktion, bevorzugt in einer Hydroformylierungsreaktion gemäß einem Verfahren wie in den Ansprüchen 4 bis 10 definiert.
